(19) Europäisches Patentamt / European Patent Office / Office européen des brevets

(11) **EP 4 636 362 A1**

(12) **EUROPEAN PATENT APPLICATION**

(43) Date of publication:
22.10.2025   Bulletin 2025/43

(21) Application number: 25170689.1

(22) Date of filing: 15.04.2025

(51) International Patent Classification (IPC):
*G01D 5/353* (2006.01)

(52) Cooperative Patent Classification (CPC):
G01D 5/35316; A61B 5/01; G01D 5/35312

(84) Designated Contracting States:
AL AT BE BG CH CY CZ DE DK EE ES FI FR GB GR HR HU IE IS IT LI LT LU LV MC ME MK MT NL NO PL PT RO RS SE SI SK SM TR
Designated Extension States:
BA
Designated Validation States:
GE KH LA MA MD TN

(30) Priority: 16.04.2024   US 202463634771 P
09.07.2024   DE 102024206477

(71) Applicant: Technische Universität Hamburg (TUUH) KöR
21073 Hamburg (DE)

(72) Inventors:
• Khatouni, Sohrab Shojaei
22299 Hamburg (DE)
• Trieu, Hoc Khiem
21394 Westergellersen (DE)

(74) Representative: RCD-Patent
Giesen, Schmelcher & Griebel
Patentanwälte PartG mbB
Kaiserstraße 100
52134 Herzogenrath (DE)

(54) **AN OPTICAL MEASUREMENT DEVICE FOR ENABLING SIMULTANEOUS MEASUREMENT OF TEMPERATURE, PRESSURE AND BENDING**

(57)   The invention pertains to an optical measurement device for enabling simultaneous measurement of temperature, pressure and bending, comprising a broadband light source (Q), a fiber (F), and an optical spectrum analyzer (OSA), whereby in the fiber (F) at least two Fiber Bragg Gratings as a Fabry-Perot Interferometer are arranged allowing for reflection of light emitted by the light source (Q) into the fiber (F), back through the fiber (F) towards the optical spectrum analyzer (OSA), whereby reflection is depending of the temperature, pressure and bending applied to the fiber (F) in the region of the Fiber Bragg Grating Fabry-Perot Interferometer, whereby by use of the at least two Fiber Bragg Gratings Fabry-Perot Interferometer additional correlation features are produced allowing for separation of temperature, pressure and bending.

The invention also pertains to a method using said optical measurement device and the uses in medicine.

Fig. 6

## Description

The invention relates to an optical measurement device for enabling simultaneous measurement of temperature, pressure and bending.

Background

Advancements in the field of microtechnology have led to a variety of novel medical devices and sensors during the last century, such as implantable cardioverter-defibrillators, contact force sensitive ablation catheters, and many more. These innovations do not only augment existing medical procedures, but also open up space for a variety of completely new, faster, more accurate and cost-effective ways to carry out treatments and diagnostics. In cases, where the addressed lesion is to be accessed through small incisions microtechnology is of particular interest. This way of procedure is referred to as minimally invasive surgery (MIS).

The minimal invasive surgery evolved following approaches to visualize internal structures such as a laparoscope.

Nowadays, endovascular MIS involves the insertion and navigation of surgical instruments through e.g. blood vessels to a lesion, often being a vessel itself.

When compared to conventional methods, MIS comes with tremendous benefits for patients such as reduced morbidity, mortality and faster convalescence, though it is also associated with many challenges and risks.

One of the challenges in all these micro-access-based examination or interventional procedures is the distorted haptic feedback that clinicians encounter from inserted instruments, which may lead to misjudgment of the actual forces acting on vasculature. This can cause clinicians to apply inappropriately high forces that increase the probability of injury or even perforation of the affected vessel.

The distortion of haptic feedback and the migration resistance encountered by surgeons increases the further the instrument is migrated into the vessel. This is due to the increasing amount of inserted surface area, upon which contact forces act in the counter direction of the intended movement. To still be able to navigate the instruments to the desired region more force needs to be applied.

Combined with the increasing resistance this may lead to elastic compression and consequently to springlike behavior of the instruments. As soon as the stored energy exceeds a certain threshold, it is often suddenly released by a leap of the distal instrument tip. Even though countermeasures such as hydrophobic coating of the instruments and special techniques are used to minimize this effect, it is still observed among clinicians and interventionists.

Guidewires, catheters or other instruments used in minimally invasive endovascular procedures are affected by this problem, causing dissection, embolism or perforation of vessels especially during endovascular navigation. The incidence increases, if patients have preexisting arterial diseases such as arteriosclerosis.

Meanwhile, there is a great demand for sensors and devices to provide additional information about the overall state of the small instruments used during coiling. Forces acting on the vasculature are of particular interest with regard to the complications of MIS in general, and coiling of intracranial aneurysm in particular.

Problem

In view of the growing demand, it is also a necessity to minimize risks associated with microvascular procedures to allow for a broader usage where classical procedures are more likely to fail. In this context it would be helpful to provide a surgeon with further details of bending, temperature and pressure in particular at the tip of a microvascular tool.

Summary of the invention

The problem is solved by an optical measurement device for enabling simultaneous measurement of temperature, pressure and bending according to claim 1. Further purposive embodiments according to the invention are subject to the dependent claims, as well as the accompanying description and figures.

Summary of the figures

In the following the invention will be explained with reference to the figures. These show

Fig. 1a          a spectrum of a transmission of an ideal Fiber Bragg Grating,

Fig. 1b          a spectrum of a reflection of the ideal Fiber Bragg Grating corresponding to Fig. 1a,

Fig. 2            an index distribution of the ideal Fiber Bragg Grating corresponding to Fig. 1a,

Fig. 3a           a spectrum of a transmission of a real Fiber Bragg Grating which may be embodied in the invention,

Fig. 3b           a spectrum of a reflection of the real Fiber Bragg Grating corresponding to Fig. 3a,

Fig. 4            an index distribution of the real Fiber Bragg Grating corresponding to Fig. 3a,

Fig. 5            exemplary grating types and their corresponding index modulations according to embodiments of the invention,

Fig. 6            a correlation between number of $\pi$-Phase-shifts and morphology of transmitted spectrum,

Fig. 7            a comparison between Fabry-Perot Interferometer (FPI) and Fiber Bragg Grating (FBG) (upper portion) and Fiber Bragg Grating Fabry-Perot Interferometer (FBGFPI) (lower portion) showing reflected power qualitatively,

Fig. 8            an exemplary RAVE element (German: Radiusvariierende Einspannvorrichtung, English: variable radius clamping device) which may be used for training of an assisted determination method,

Fig. 9            an exemplary setup which may be used for training of an assisted determination method, and

Fig. 10 and 11   exemplary embodiments and uses according to the invention.

Detailed description

The present disclosure describes preferred embodiments with reference to the figures, in which like reference signs represent the same or similar elements. Reference throughout this specification to "one embodiment," "an embodiment," or similar language means that a particular feature, structure, or characteristic described in connection with the embodiment is included in at least one embodiment of the present invention. Thus, appearances of the phrases "in one embodiment," "in an embodiment," and similar language throughout this specification may, but do not necessarily, all refer to the same embodiment.

The described features, structures, or characteristics of the invention may be combined in any suitable manner in one or more embodiments. In the description, numerous specific details are recited to provide a thorough understanding of embodiments of the invention. I.e., unless indicated as alternative, only a feature of an embodiment may also be utilized in another embodiment.

The phrases "at least one", "one or more", and "and/or" are open-ended expressions that are both conjunctive and disjunctive in operation. For example, each of the expressions "at least one of A, B and C", "at least one of A, B, or C", "one or more of A, B, and C", "one or more of A, B, or C" and "A, B, and/or C" means A alone, B alone, C alone, A and B together, A and C together, B and C together, or A, B and C together. When each one of A, B, and C in the above expressions refers to an element, such as X, Y, and Z, or class of elements, such as X1-Xn, Y1-Ym, and Z1-Zo, the phrase is intended to refer to a single element selected from X, Y, and Z, a combination of elements selected from the same class (e.g., X1 and X2) as well as a combination of elements selected from two or more classes (e.g., Y1 and Zo).

The term "a" or "an" entity refers to one or more of that entity. As such, the terms "a" (or "an"), "one or more" and "at least one" can be used interchangeably herein. It is also to be noted that the terms "comprising", "including", and "having" can be used interchangeably. The preceding is a simplified summary of the disclosure to provide an understanding of some aspects of the disclosure. This summary is neither an extensive nor exhaustive overview of the disclosure and its various aspects, embodiments, and configurations. It is intended neither to identify key or critical elements of the disclosure nor to delineate the scope of the disclosure but to present selected concepts of the disclosure in a simplified form as an introduction to the more detailed description presented below. As will be appreciated, other aspects, embodiments, and configurations of the disclosure are possible utilizing, alone or in combination, one or more of the features set forth above or described in detail below.

For purposes of explanation, numerous details are set forth in order to provide a thorough understanding of example embodiments. It should be appreciated however that the techniques herein may be practiced in a variety of ways beyond the specific details set forth herein.

Furthermore, while the exemplary embodiments illustrated herein may show the various components of the system collocated, it is to be appreciated that the various components of the system can be located at distant portions of a distributed network, such as a communications network and/or the Internet, or within a dedicated secure, unsecured and/or encrypted system. Thus, it should be appreciated that the components of the system can be combined into one or more devices, or collocated on a particular node/element(s) of a distributed network, such as a communications network. As will be appreciated from the description, and for reasons of computational efficiency, the components of the system can be arranged at any location within a distributed network without affecting the operation of the system.

Furthermore, it should be appreciated that the various links, including communications channel(s), connecting the elements (which may not be not shown) can be wired or wireless links, or any combination thereof, or any other known or later developed element(s) that is/are capable of supplying and/or communicating data and/or signals to and from the connected elements. The term module as used herein can refer to any known or later developed hardware, software, firmware, or combination thereof that is capable of performing the functionality associated with that element. The terms determine, calculate and compute, and variations thereof, as used herein are used interchangeably and include any type of methodology, process, mathematical operation or technique.

While flowcharts/operational flows have been discussed in relation to a particular exemplary sequence of events, it should be appreciated that changes to this sequence can occur without materially effecting the operation of the embodiment(s). Additionally, the exact sequence of events need not occur as set forth in the exemplary embodiments, but rather the steps can be performed by one or the other device(s) in the system. Additionally, the exemplary techniques illustrated herein are not limited to the specifically illustrated embodiments but can also be utilized with the other exemplary embodiments and each described feature is individually and separately claimable.

As will be appreciated by one skilled in the art, aspects of the present disclosure may be embodied as a system, method, and/or computer program product. Thus, aspects of the present disclosure may be embodied entirely in hardware, entirely in software (including, but not limited to, firmware, program code, resident software, microcode), or in a combination of hardware and software. All such embodiments may generally be referred to herein as a circuit, a module, or a system. In addition, aspects of the inventive concepts may be in the form of a computer program product embodied in one or more computer readable media having computer readable program code embodied thereon.

A computer readable medium as described herein may be a computer readable storage medium, examples of which include, but are not limited to, an electronic, magnetic, optical, electromagnetic, infrared, or semiconductor system, apparatus, or device, or any suitable combination thereof. As used herein, a computer readable storage medium may be any non-transitory, tangible medium that can contain or store a program for use by or in connection with an instruction execution system, apparatus, device, computer, computing system, computer system, or any programmable machine or device that inputs, processes, and outputs instructions, commands, or data. A non-exhaustive list of specific examples of a computer readable storage medium include an electrical connection having one or more wires, a portable computer diskette, a floppy disk, a hard disk, a random access memory (RAM), a read-only memory (ROM), a USB flash drive, an non-volatile RAM (NVRAM or NOVRAM), an erasable programmable read-only memory (EPROM or Flash memory), a flash memory card, an electrically erasable programmable read-only memory (EEPROM), an optical fiber, a portable compact disc read-only memory (CD-ROM), a DVD-ROM, an optical storage device, a magnetic storage device, or any suitable combination thereof. A computer readable storage medium can be any computer readable medium that is not a computer readable signal medium such as a propagated data signal with computer readable program code embodied therein.

Program code may be embodied as computer-readable instructions stored on or in a computer readable storage medium as, for example, source code, object code, interpretive code, executable code, or combinations thereof. Any standard or proprietary, programming or interpretive language can be used to produce the computer-executable instructions. Examples of such languages include C, C++, C#, Pascal, JAVA, JAVA Script, BASIC, Smalltalk, Visual Basic, and Visual C++.

Transmission of program code embodied on a computer readable medium can occur using any appropriate medium including, but not limited to, wireless, wired, optical fiber cable, radio frequency (RF), or any suitable combination thereof.

The program code may execute entirely on a user's/operator's/administrator's computer, partly on such a computer, as a stand-alone software package, partly on the user's/operator's/administrator's computer and partly on a remote computer, or entirely on a remote computer or server. Any such remote computer may be connected to the user's/operator's/administrator's computer through any type of network, including a local area network (LAN) or a wide area network (WAN), or the connection may be made to an external computer (for example, through the Internet using an Internet Service Provider).

Additionally, the systems, methods and protocols described herein can be implemented to improve one or more of a special purpose computer, a programmed microprocessor or microcontroller and peripheral integrated circuit element(s), an ASIC or other integrated circuit, a digital signal processor, a hard-wired electronic or logic circuit such as discrete element circuit, a programmable logic device such as PLD, PLA, FPGA, PAL, a smartphone, any comparable means, or the like. In general, any device capable of implementing a state machine that is in turn capable of implementing the methodology illustrated herein can benefit from the various communication methods, protocols and techniques according to the disclosure provided herein.

Examples of the processors as described herein include, but are not limited to, at least one of Qualcomm® Snapdragon® 800 and 801, Qualcomm® Snapdragon® 610 and 615 with 4G LTE Integration and 64-bit computing, Apple® A7, A8, A8X, A9, A9X, or A10 processors with 64-bit architecture, Apple® M7, M8, M9, or M10 motion coprocessors, Samsung® Exynos® series, the Intel® Core™ family of processors, the Intel® Xeon® family of processors, the Intel® Atom™ family of processors, the Intel Itanium® family of processors, Intel® Core® i5-4670K and i7-4770K 22nm Haswell, Intel® Core® i5-3570K 22nm Ivy Bridge, the AMD® FX™ family of processors, AMD® FX-4300, FX-6300, and FX-8350 32nm Vishera, AMD® Kaveri processors, ARM® Cortex™-M processors, ARM® Cortex-A and ARM926EJ-S™ processors, Broadcom® AirForce BCM4704/BCM4703 wireless networking processors, the AR7100 Wireless Network Processing Unit, other industry-equivalent processors, and may perform computational functions using any known or future-developed standard, instruction set, libraries, and/or architecture.

Furthermore, the disclosed methods may be readily implemented in software using object or object-oriented software development environments that provide portable source code that can be set on a variety of computer, workstation or mobile device platforms, e.g., smartphones or mobile phones or vehicles. Alternatively, the disclosed system may be implemented partially in hardware using standard logic circuits or a VLSI design. Whether software or hardware is used to implement the systems in accordance with embodiments is dependent on the speed and/or efficiency requirements of the system, the particular function, and the particular software or hardware systems or microprocessor or microcomputer systems being utilized. The methods illustrated herein however can be readily implemented in hardware and/or software using any known or later developed systems or structures, devices and/or software by those of ordinary skill in the applicable art from the functional description provided herein and with a general basic knowledge of the computer and image processing arts.

Moreover, the disclosed methods may be readily implemented in software executed on programmed general-purpose computer, a special purpose computer, mobile device, smartphone, a microprocessor, or the like. In these instances, the systems and methods of inventive concepts can be implemented as program embedded on personal computer such as JAVA® or CGI script, as a resource residing on a server or graphics workstation, as a routine embedded in a dedicated image processing system, as a plug-in, or the like. The system can also be implemented by physically incorporating the system and method into a software and/or hardware system, such as the hardware and software systems of an image processor.

While this technology has been described in conjunction with a number of embodiments, it is evident that many alternatives, modifications and variations would be or are apparent to those of ordinary skill in the applicable arts. Accordingly, it is intended to embrace all such alternatives, modifications, equivalents, and variations that are within the spirit and scope of this disclosure.

While being described with reference to a medical application in the following, the invention is not limited thereto.

In particular, we will now describe the invention with reference to a medical device based on an ultrathin fiber containing micro-optomechanical sensors intended to be used in intracranial endovascular minimally invasive surgery. The device i- also referred to as TacFiber - may be designed for usage in the smallest cranial vessels that are currently being treated with microcoils.

I.e., the device may be offering other dimensions for other usages.

Furthermore, while not a necessity for the invention, the invented device may also be designed to be employed in a magnetic resonance imaging (MRI) environment, enabling possible future applications using metal-free microcoils.

The device is based on Fiber Bragg Gratings (FBGs) that are embedded in a single mode fiber, in particular a silicon dioxide single mode fiber.

Standard FBGs response to strain (i.e. forces or pressures) due to the photo-elastic effect as well as to temperatures due to the thermo-photo effect in a similar way which causes cross sensitivity which makes it very difficult to distinguish both measurands simultaneously. Bending also affects the grating pitch of the FBG hence contributing to another cross sensitivity.

By multiplexing several gratings at specific locations of the fiber, strain, temperature and bending can be distinguished and simultaneously quantified.

Particularly, the system may work if the distance is calculated according to equations in the dissertation and tuned in the way that the transmission bands (or wavelenghts) of the the Fabry Perot Interferometer (which is the superstructure of the two FBGs that have the same center wavelength) and there are at least 2 more transmissions bands (wavelenths) in the reflection band of the FBG - so in total there are 3 or more transmission bands in the reflection band of the FBG.

By interference of at least two FBGs (resulting e.g. in a FBGFPI) additional features as subpeaks in the spectrum of reflection are generated.

This approach of feature engineering enables the generation of enriched signal patterns with slightly different response to strain, temperature and bending.

Here Machine Learning has been proven to distinguish these three measurands simultaneously by analyzing the enriched features.

One specific feature of the invention is to design the reflectivity of the two mirrors of the FPI in such a way that the power of the reflected wave from mirror 1 and the power of the transmitted fraction from mirror 1 will match each other after this transmitted wave has been reflected at mirror 2. I.e. The power of the transmitted wave with the fraction (1-R1), where R1 is the reflectivity of mirror 1, will be further reduced by the factor R2, where R2 is the reflectivity of mirror 2. This resulting power R2*(1-R1) should match the power R1 of the reflected wave at mirror 1.

Getting these two powers closed to each other enhances the interference of the two signals. Ideally, the two values should match each other. But being closed to each other also work properly, as shown in our tested design with R1 = 45% and R2 = 75%.

While the wave reflected at mirror 1 still has 45% of its original power, 55% of the power is transmitted and then finally get reflected from mirror 2 with a reflectivity of 75%. I.e. this second reflected wave shows about 41% of its original power, which is closed to the 45% of the reflected wave at mirror 1. Requiring no perfect matching for good operation is essential for practical use as manufacturing tolerances can create minor deviations.

In other words, the reflectivity of a first Fiber Bragg Grating and a second Fiber Bragg Gratings is selected such that the power being reflected by the second Fiber Bragg Grating after having been transmitted via the first Fiber Bragg Grating is within the same scale as the reflected power from the first Fiber Bragg Grating, i.e. they diverge by approx. 10 % or less.

This feature goes beyond the state of the art for FBGFPI which utilizes two highly reflective mirrors with very high reflectivity of 95% and above in order to obtain a reflection spectrum with sharp transmission peaks. In contrast to these sharp transmission peaks our feature engineering approach targets the generation of enriched features in the spectrum which are not available in the sharp peaks.

In addition, an exemplary size of approximately 170 $\mu$m allows for using the device even in a variety of small vessels such as in peripheral or cerebral vasculature. Unlike other instruments the invention provides information about the compression of the fiber by measuring the pressure even in multiple locations along the longitudinal axis. This feature is particular useful whenever the path between the incision and the lesion, that is being addressed, is long and leads through tortuous vessels. Strong compression can be detected early, thereby minimizing the probability of an unintentional sudden movement of the instrument's tip.

The device may additionally also be designed to only use non-ferromagnetic materials to be suitable for magnetic resonance imaging (MRI) applications. The sensor performance is also not affected by radio waves on any kind due to its optical measurement principle.

Also, the invention may be used as delivery wire or pusher wire, when attached to an interlock at its distal tip.

In further contrast to conventional methods of signal evaluation for Fiber Bragg Grating (e.g. peak detection), in this invention machine and deep learning approaches may be used to evaluate the sensor signal.

With this approach the richness and information density of the reflected spectrum is conserved and signal features that were otherwise hard to detect are extracted.

It is noted that further information, in particular with respect to medical background, may be found in the dissertation of the inventor Sohrab Shojaei Khatouni entitled "TacFiber: A Micro-Optomechanical Sensor for Simultaneous Measurement of Axial Forces, Temperature and Curvature in Intracranial Endovascular Minimally Invasive Surgery" in Section 2.1. Furthermore, the mechanics experienced by an intravascular device are discussed in Section 2.2. Also, the fundamentals of fiber optics are touched in section 2.3.

In particular, this invention references section 2.4 for more details on optical Fiber Bragg Gratings as well as section 2.5 of the aforementioned dissertation.

These sections are included by way of explicit reference.

The invention is based on the fact, that whenever a fiber is subjected to external forces, these forces lead to elastic or plastic deformation. In the context of the invention only elastic deformations are considered. Any elastic deformation influences the lattice constant of microstructure constituting the fiber which in turn effects the refractive index n of the fiber.

The following equation describes the strain dependency of the directional relative permittivity of a material, also known as dielectric constant:

$$\Delta \left( \frac{1}{\overline{\varepsilon_r}} \right) = \begin{bmatrix} \rho_{11} & \rho_{12} & \rho_{12} \\ \rho_{12} & \rho_{11} & \rho_{12} \\ \rho_{12} & \rho_{12} & \rho_{11} \end{bmatrix} \cdot \begin{bmatrix} \varepsilon_x \\ \varepsilon_y \\ \varepsilon_z \end{bmatrix},$$

whereby $\rho_{11} \neq \rho_{12}$ and $\rho_{11}$, $\rho_{12}$ not being constant. It is noted that both $\rho_{11}$ as well as $\rho_{12}$ are subject to dispersion, i.e. they are wavelength-dependent.

Furthermore, the invention is also based on the fact, that whenever a fiber is subjected to a temperature, the temperature affects the refractive index n of the fiber as well. However, unlike the photo-elastic-effect, the thermo-photo-effect is isotropic and mostly dependent on the used medium.

When an electro-magnetic wave passes the boundary between two media with different refractive indexes, the wave is partially reflected and transmitted. This effect is utilized in Fiber Bragg Grating sensors to cause wavelength selective transmission and reflection by periodic refractive index modulations along the longitudinal axis of a waveguide. These properties of Fiber Bragg Gratings are discussed by the means of coupled mode theory (CMT) in the following.

To briefly illustrate the working principle in a few steps, the phase constant β of an electromagnetic wave with wavelength $\lambda_x$ that propagates through fiber is considered:

$$\beta = \frac{2\pi}{\lambda_x} \cdot n_{eff}$$

The phase constant β is measured in radians per unit distance. Hence, it is a good parameter to assess the change in phase of a plane wave for a given traveled distance. According to CMT the momentum of the original electromagnetic wave, which is defined as the cross product of the electric field $\vec{E}$ and the magnetic field $\vec{H}$, is preserved after refraction and reflection due to energy preservation. Considering transversal electromagnetic waves (TEM-Waves) that propagates with negligible loss the wave number k is equal to the phase constant β. For energy to be transferred from the forward propagating mode with $\beta_f$ to the backward propagating mode with $\beta_b$, the backward propagating electromagnetic waves that are generated at each perturbation zone have to be in phase.

If one assumes two pertubation zones $p_0$ and $p_1$ in a distance $\pi$ and two reflected electromagnetic waves with phase constants $\beta_{b0}$ and $\beta_{b1}$ one may evaluate the mismatch at boundary $p_0$. Therewith the value of the phase constant $\beta_{b1}$ of the wave that is generated at boundary p1 can be expressed as the sum of the phase constant of the driving mode $\beta_f$ with negative sign (because of the reflection) and a phase shift that is the phase factor of the disturbance. The mismatch between the phase $\beta_{b0}$ of the back propagating mode generated at p0 and the phase $\beta_{b1}$ of the back propagating mode generated at p1 can be expressed with following term:

$$\Delta\beta = \beta_{b0} - \beta_{b1}$$

The phase factor is determined by the path $\Lambda$ between the two boundaries p0 and p1.

$$\beta_{b1} = -\left(\beta_f - \frac{2\pi}{\Lambda}\right)$$

Which can be reintroduced into the above formula while setting the mismatch $\Delta\beta$ = 0:

$$0 = \beta_{b0} + \left(\beta_f - \frac{2\pi}{\Lambda}\right)$$

Since the generated wave at boundary p0 is a reflection of the forward propagating mode, its phase $\beta_{b0} = \beta_f$ at p0, leading to

$$\beta_f = \frac{\pi}{\Lambda}$$

and finally, to

$$\lambda_x = 2n_{eff}\Lambda$$

Therefore, when the wavelength $\lambda_x$ of an electromagnetic waves satisfies the above equation in a fiber with A-periodic perturbations, the generated co-propagating backward modes are in phase, allowing energy transfer between the forward mode and the backward mode for this particular wavelength. This coherently reflected wavelength, at which there is no phase mismatch, is denoted as Bragg wavelength $\lambda_B$.

The effective refractive index $n_{eff}$ for $SiO_2$ in the above equations may be given by

$$n_{eff} \approx n_{CL} + \left(1.1428 - \frac{0.996}{\upsilon}\right)^2 \Delta n,$$

whereby the following values may be assumed $\upsilon \approx 2$, $n_{CL} \approx 1.445$ and $\Delta n \approx 0.01$ at room temperature, which leads to $n_{eff} \approx$

1.450.

For other fiber types the values may vary.

Only a small portion of the electromagnetic wave energy is reflected at a single boundary interface for reasonably small refractive index changes, allowing for most of the energy to pass boundary interfaces. To achieve high wavelength selective reflection, multiple small refractive index changes shall be induced periodically along the propagation path of an electromagnetic wave. The summation of all coherent reflections ultimately leads to wavelength selective reflection. The reflectivity can be tuned by changing the number of perturbations. Moreover, phase shifts that are multiples of $2\pi$ generate higher order responses.

This effect is used in Fiber Bragg Gratings, where multiple periodic refractive index perturbations are induced along the longitudinal axis of a single-mode waveguide. In the following, the pitch of the perturbations is denoted as A, with the value $\dfrac{\lambda_B}{2n_{eff}}$ with $\lambda$ being the wavelength that must be reflected.

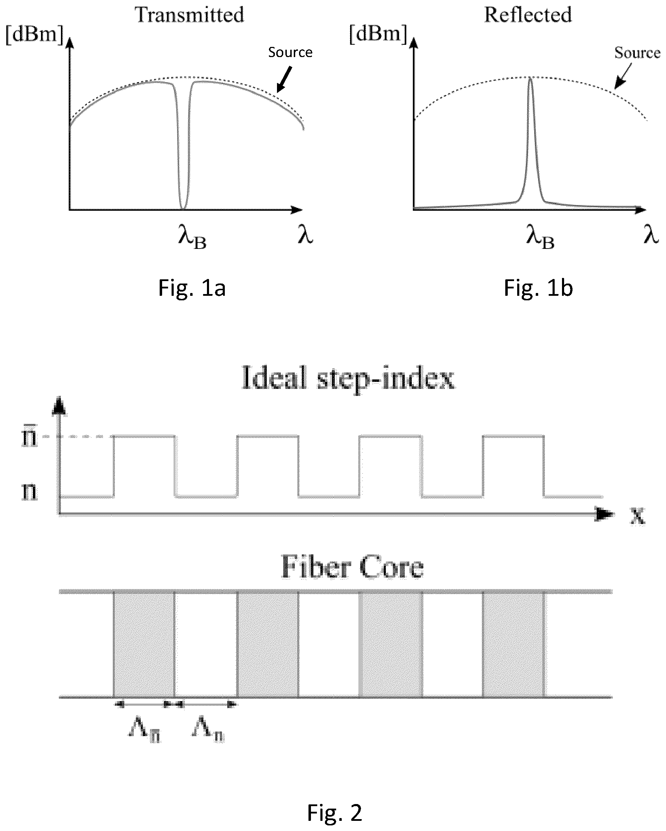

In theory, using a broadband source and an optical spectrum analyzer (OSA) to assess the transmission and reflection properties of an ideal Fiber Bragg Grating, the reflected spectrum would show a sharp peak at $\lambda_B$, while the same wavelength would be missing in the transmitted spectrum, as shown in Figure 1a and 1b and assuming an ideal step-index Fiber Grating as shown in Figure 2.

To also take higher order coherent interference into account, a more comprehensive form is used throughout this work with $\lambda_B$ denoting reflected wavelength, $\Lambda_G$ grating pitch, $n_{eff}$ for the effective refractive index, and finally m denoting the harmonic order number that is 1 for Bragg resonance.

$$\Lambda_G = \frac{m\lambda_B}{2n_{eff}}.$$

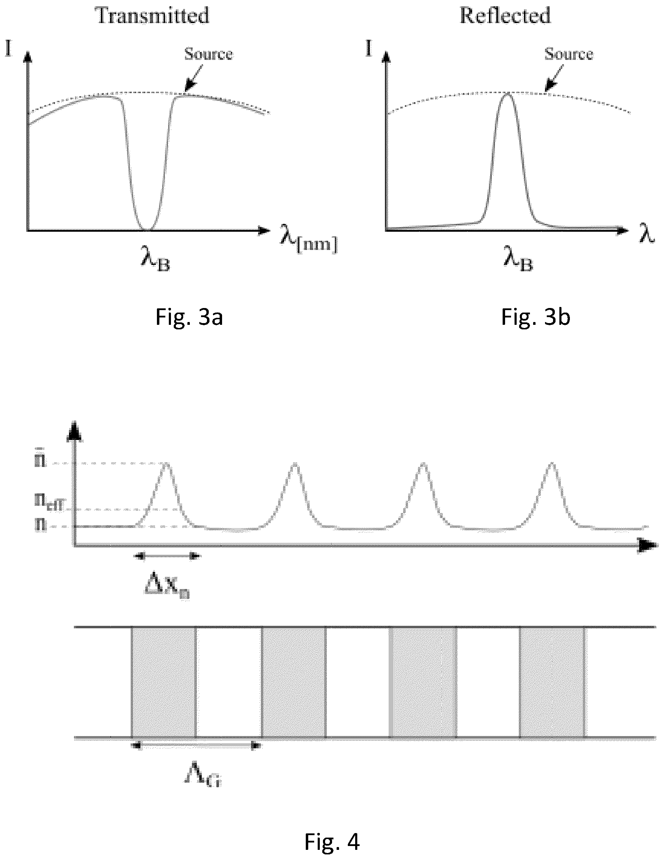

However, the ideal may not be achieved easily. Therefore, we assume a representation of a real Fiber Bragg Grating as shown in Figures 3a, 3b and Figure 4.

Grating length: In theory both, high refractive index zones (HRIZ) and low refractive index zones (LRIZ) contribute to overall coherent reflection. To satisfy the Bragg condition both zones shall be fabricated with precise length that match corresponding index values. The LRIZ are already in place in regular Ge doped single-mode $SiO_2$ fibers that come with sufficiently uniform refractive index cores. However, the challenging part is to inscribe uniform HRIZ with precise length into this substrate. A common way to circumvent this challenge is to eliminate the contribution of the HRIZ to overall reflection by decreasing the length $\Lambda_{\overline{n}}$ of these zones to a minimum $\Delta x_{\overline{n}} \ll \Lambda_{\overline{n}}$. Thereby the HRIZ still lead to reflection, which is paramount for the grating to satisfy the Bragg condition, but does not contribute to the coherent reflection. The coherent reflection is then achieved by modifying the length $\Lambda_n$ of the LRIZ accordingly to satisfy $\Delta x_{\overline{n}} + \Lambda_n = \Lambda_G$ with $\Lambda_G$ satisfying the Bragg condition.

Refractive index: In the same way, for both zones to contribute to coherent reflection, the refractive indexes shall be fabricated in a precise and uniform way with step like changes between two zones. Setting the length of the HRIZ to $\Delta x_{\overline{n}} \ll \Lambda_{\overline{n}}$ eliminates this requirement. An overall effective refractive index $n_{eff}$ which is determined for the length of the Fiber-Bragg-Grating is introduced instead of individual refractive indexes $\overline{n}$ and n for individual zones. The effective refractive index is then used to determine the overall grating pitch $\Lambda_G$.

Even though the changes in refractive index along the grating length $\Delta x_n$ lead to multiple reflections at different locations along the zone, this approach is successfully used for selective reflection of $\lambda_B$, as shown in Figure 3a and 3b.

The reflected spectrum is shaped like a Gaussian bell, instead of an ideal peak, that decreases in width with decreasing $\Delta x_{\overline{n}}$ and its maximum at $\lambda_B$.

The grating pitch $\Lambda_G$ is calculated by following equation:

$$\Lambda_G = \frac{\lambda_B}{2n_{eff}}$$

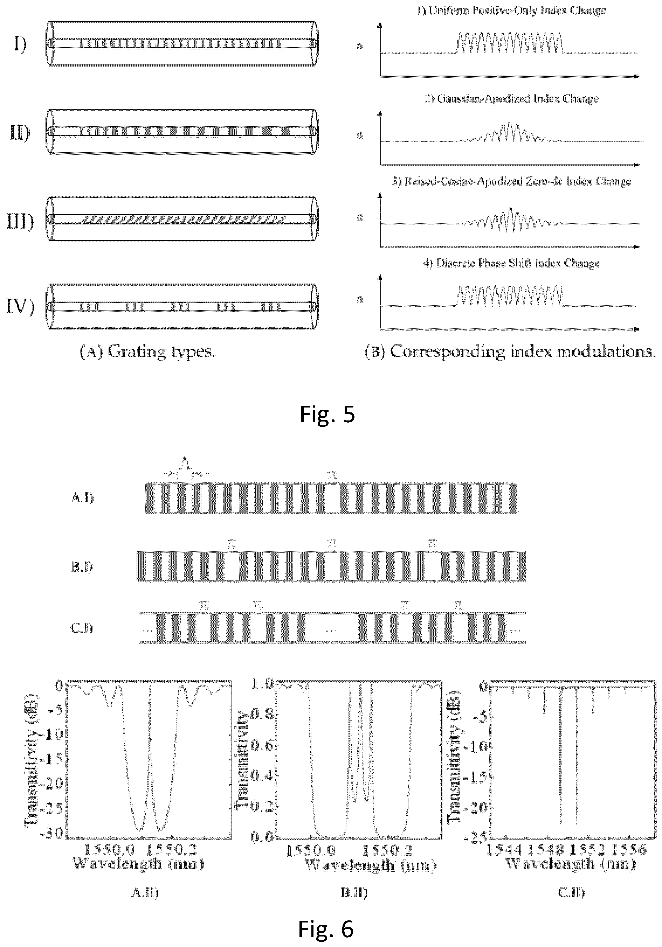

Since the discovery of Fiber Bragg Gratings, there were many ways suggested how to modify the spectral response and overall behavior of the units by varying the design parameters of the gratings. The most fundamental ways of modification are illustrated in Figure 5. Basically, the grating I) in the left portion refers to a uniform grating, whereas the grating II) is chirped. The grating III) is tilted and grating IV) represents a superstructure. On the right portion of Figure 5 the corresponding changes in the refractive index are shown.

The length of the grating might have a direct impact on the reflectivity. When the length of the grating is increased, the reflectivity increases as well. On the other hand, the spatial expansion of a grating represents its sensing area. Therefore, in applications where large areas must be monitored (structural health) and there is no need for high local resolution, the grating length is often increased, increasing the sensing area, which in turn decreases the sensitivity. In contrast, for high spatial resolutions grating length needs to be short.

The type of the grating determines the shape of the reflected spectrum, in particular the peak. The narrow-reflected peak of uniform gratings is broadened with chirped gratings. This kind of grating can be used as band-pass or band-stop filter. Tilted gratings are predominantly used in multi-mode fibers, to excite cladding modes. The type of the index perturbation also effects the shape of the reflected spectrum with respect to the side lobes. The most common way to modify the index perturbation is by Gaussian apodization, which increases the SLSR (side lobe suppression ratio), suppressing side lobes.

Finally, gratings can be augmented by superstructures, hence added periodic grating elements resulting in overlapping structures, or periodic blank spots, like in sampled Fiber Bragg Gratings. In overlapping structures, the reflected spectrum is typically a superposition of the individual reflected spectra with some interference. For instance, a long-period grating (LPG) and a Fiber Bragg Grating can be overlapped to increase the information density of the transmitted spectrum.

This technique is used in multi-mode fibers and applications, where transmitted signal energy can be retrieved. For single-mode applications, the sampled super-structures are more relevant, shown in Figure 5 IV), and the corresponding index perturbation. In contrast to overlapping gratings, sampled or shifted Fiber Bragg Gratings result in a set of sub-gratings.

Depending on the number and spatial separation d of sub-gratings, SFBGs (superstructure Fiber Bragg Gratings) are denoted as follows:

1. sampled FBG (d < π < A/2)
2. π-shifted FBG (d = π = A/2)
3. Fabry-Perot Fiber Bragg Grating (FPFBG) (d ≪ 2π)
4. cascaded FPFBG

These types can of course be mixed to generate a superposition of the properties of each type of superstructure.

By tuning the sampling length as well as duty cycle of the laser and number of samples, different reflection pattern can be generated. The π - phase - shifted Fiber Bragg Gratings are generated by splitting a uniform Fiber Bragg Grating into two or more identical sub- Fiber Bragg Gratings with a half-pitch phase difference between them.

Conceptually, any superstructure Fiber Bragg Grating is a concatenation of Fabry-Perot interferometers based on the Fabry-Perot effect. The space between the individual sub-gratings can be considered as a Fabry-Perot cavity. Therefore, single shift SFBGs are referred to as FPFBGs, Grating Fabry Perot cavity, Fiber Bragg Grating Fabry-Perot Cavity, or FBGFPI. From here, the term FBGFPI is used to refer to this type of superstructure.

In general, a Fabry-Perot cavity is generated with two highly reflective parallel mirrors (R = 99%) that are facing one another, separated by the length $L_{FP}$. Depending on $L_{FP}$, there is a set of wavelengths that are fully transmitted by this setup.

The initial electromagnetic wave $E_{in}$ is transmitted through a first mirror, almost fully reflected by a second mirror to then bounce back and forth in the cavity. Each time an electromagnetic wave hits the second mirror, a small portion of its energy is transmitted through the second mirror until is almost fully transmitted. To illustrate the math behind the Fabry-Perot effect, the indexes of the fields are appended with a t whenever they are a result of a transmitted field. Similarly, when a field is a result of a reflection, its index is appended with a r. If multiple subsequent reflections are considered, the reflection indexes are abbreviated with corresponding number of reflections. For instance, 6 time reflected fields ($E_{rrrrrr}$) are denoted as $E_{6r}$. Therefore, the strength of the first field, that has been transmitted through the first mirror, and reflected 3 times subsequently is denoted as $E_{t3r}$. The intensity reflectivity R and transmission T are the square roots of the corresponding field reflectivity r and transmission t values, i.e. $t^2 = T = 1 - R = 1 - r^2$.

A transmitted field strength is the product of the original field and t. As a result, the first transmitted field is expressed as $E_t = tE_{in}$ and the reflected field is expressed as $E_r = rE_{in}$ while at the second mirror the transmitted field will be $Ett = tE_t e^{i\kappa L_{FP}}$ since the phase of the field is just shifted by the phase $\kappa L_{FP}$ while traveling through the cavity. Likewise, the reflected portion at the second mirror will be $E_{tr} = t E_{in} e^{i\kappa L_{FP}} r$. The reflected part of the field then propagates back to first mirror, shifting its phase again $E_{tr} = t E_{in} e^{i\kappa L_{FP}} r e^{i\kappa L_{FP}}$. Considering the part of the field that is reflected from the first mirror subsequently, the strength of the field can be expressed as $Et_{2r} = t E_{in} e^{i\kappa L_{FP}} r e^{i\kappa L_{FP}} r$. This field propagates back to the second mirror, where its phase is again shifted $\kappa L_{FP}$, leading to

$$E_{t2r} = t\, E_{in} e^{i\kappa L_{FP}}\, r\, e^{i\kappa L_{FP}}\, r\, e^{i\kappa L_{FP}} = t\, E_{in} e^{i\kappa L_{FP}} \left( r\, e^{i\kappa L_{FP}} \right)^2 = t\, E_{in} e^{i\kappa L_{FP}} \left( r^2\, e^{2i\kappa L_{FP}} \right).$$

Each time the field bounces back and forth between the mirrors, it is multiplied by the term ($r^2 e^{2i\kappa L_{FP}}$). As the bounced back field incidents the second mirror, a portion of it is transmitted.

As a consequence, the total amount of the transmitted field is the sum of sub-transmissions that occur at the second mirror:

$$E_{out} = t^2\, e^{i\kappa L_{FP}} E_{in} \sum_{n=1}^{\infty} \left(r^2\, e^{2i\kappa L_{FP}}\right)^n = (1-R)e^{i\kappa L_{FP}} E_{in} \sum_{n=1}^{\infty} \left(R\, e^{2i\kappa L_{FP}}\right)^n$$

The closed form solution of the geometric sum in the equation above is

$$E_{out} = (1-R)e^{i\kappa L_{FP}} E_{in}\, \frac{1}{1-(R\, e^{2i\kappa L_{FP}})}$$

As result, the field $E_{out}$ that is transmitted through the Fabry-Perot cavity is a function of the incidence field $E_{in}$, the reflectivity of the mirrors and the length of the cavity $L_{FP}$. The field transmission through both mirrors is defined as the ratio of the output field $E_{out}$ and the incidence field $E_{in}$:

$$\frac{E_{out}}{E_{in}} = \frac{(1-R)e^{i\kappa L_{FP}} E_{in}\, \frac{1}{1-(R\, e^{2i\kappa L_{FP}})}}{E_{in}} = \frac{(1-R)e^{i\kappa L_{FP}}}{1-(R\, e^{2i\kappa L_{FP}})}$$

Likewise, the square of the magnitude of the field transmission is the transmission intensity

$$\mathbb{T} = \frac{I_{out}}{I_{in}} = \left|\frac{E_{out}}{E_{in}}\right|^2 = \frac{1}{1 + \frac{4R}{(1-R)^2}(\sin(\kappa L_{FP}))^2}$$

Finally, if we demand the phase $\kappa$ to be the phase constant $\beta$ as introduced in the beginning, one may substitute $\kappa = \frac{2\pi n}{\lambda}$.

Upon closer inspection, it becomes evident that for a high reflectivity $R \approx 1$ the transmission T is a very small number close to zero for most wavelength $\lambda$. Only when the argument of the $\sin^2$-term is a multiple of $\pi$, the transmission becomes 1.

The finesse of a Fabry-Perot cavity $\mathfrak{F}$ is defined as measure of its selectivity. A high Finesse corresponds to narrow transmission peaks:

$$\mathfrak{F} = \frac{\pi\sqrt{R}}{1-R}$$

The free spectral range (FSR) is another useful parameter to characterize FPIs separation between the transmission peaks in terms of wavelength:

$$FSR_{\Delta\lambda} = \frac{\lambda^2}{2L_{FP}n_g}$$

Therefore, the distance between the transmissions peaks is dependent on the considered wavelength $\lambda$ in free-space (aka. free-space wavelength), the cavity length $L_{FP}$, and the group refractive index $n_g$. In in-fiber FPIs, where the reflectors are constituted by two Fiber-Bragg Gratings, the group refractive index $n_g = n_{eff}$.

The cavity length consists of the effective length of the gratings $L_{eff.g1}$, $L_{eff.g2}$, and the spatial distance between the grating $d_{g12}$, i.e. $L_{FP} = L_{eff.g1} + L_{eff.g2} + d_{g12}$.

The effective grating length can be expressed as a function of its physical length $L_g$ and reflectivity R, i.e.

$$L_{eff} = L_g \frac{\sqrt{R}}{2 \tanh^{-1} \sqrt{R}}.$$

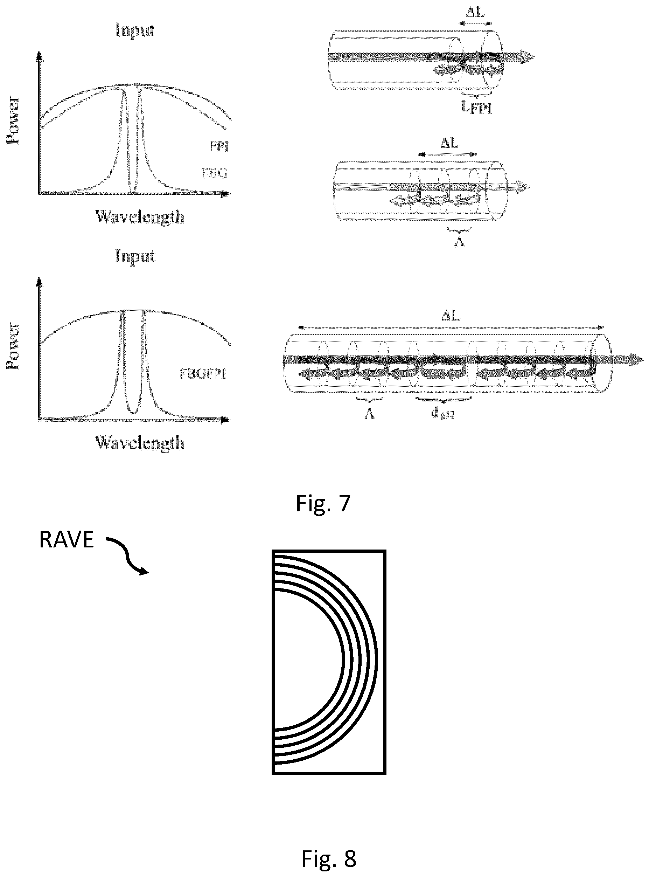

As a consequence, varying the reflectivity of the Fiber Bragg Gratings impacts the FSR (free spectral range). Taking this approach to an extreme, a Gires-Tournois Interferometer (GTI) all-pass filter can be generated that typically consists of a low reflective Fiber Bragg Grating and an almost totally reflective Fiber Bragg Grating. GTIs are used to generate multi, near identical channel dispersion compensators. By introducing different optical path lengths for various wavelengths, the compensators effectively balance the dispersion effects in multiple adjacent optical communication channels. Figure 7 illustrates the contrast between the filter properties of an FPI and a Fiber Bragg Grating. The graphs show the reflected power qualitatively, plotted against the corresponding wavelength.

In conclusion, when broadband optical radiation interacts with a configuration comprising two or more Fiber Bragg Gratings, multiple FPIs may be established. These FPIs function as wavelength-selective filters, producing transmission windows for particular wavelengths within the reflection spectrum of the Fiber Bragg Gratings.

The number of pass-bands N within the reflection band, represented by $\Delta\lambda_{FBG}$, can be estimated by dividing the reflection band by the free spectral range $FSR_{\Delta\lambda}$ channel spacing:

$$N_{PB} = \frac{\Delta\lambda_{FBG}}{FSR_{\Delta\lambda}} = \frac{2n_{eff}L_{FP}\Delta\lambda_{FBG}}{\lambda^2}$$

In the given equation, the reflection band $\Delta\lambda_{FBG}$, denotes the spectral width of the reflection spectrum of a Fiber Bragg Grating, not to be confused with the Bragg wavelength shift $\Delta\lambda_B$.

One of main advantages of Fiber Bragg Grating is the possibility of multiplexing, which means having multiple sensors in a single fiber. There are three common ways of multiplexing in Fiber Bragg Grating:

- wavelength-division multiplexing (WDM): multiple Fiber Bragg Gratings with different central wavelength in a single core fiber. The sensor signal is generated by a broadband source and demodulated by assigning Fiber Bragg Grating to their according bandwidth,
- time-division multiplexing (TDM): multiple Fiber Bragg Gratings with almost identical central wavelength in a single core fiber. The sensor signal is generated by a pulsed source and demodulated by a high-speed detector, that assigns signals to a Fiber Bragg Grating location according to their respective variations in signal runtime that are caused by the spatial distribution of the Fiber Bragg Gratings,

- frequency-shifted interferometry (FSI): multiple Fiber Bragg Gratings with almost identical central wavelength in a single core fiber. The sensor signal is generated by an adjustable semiconductor laser and demodulated by a series of optical devices such as circulators, couplers and acousto-optic modulators in a way that spatial information is retrieved by analyzing signal phase properties,
- space division multiplexing (SDM): multiple Fiber Bragg Gratings with almost identical central wavelength in a multicore fiber. The sensor signal is generated by a super-continuum light source and demodulated by a filament splicer in a way that individual cores can be addressed by alignment,
- optical time domain reflector (OTDR): multiple Fiber Bragg Gratings with almost identical central wavelength in a single core fiber. The sensor signal is generated by a fiber-optic reflectometer. A probe pulse is first introduced in the sensor fiber. As a result, a series of time-separated pulses are reflected that are subsequently introduced in a reference fiber with two Fiber Bragg Grating with central wavelength below and above that of the sensor Fiber Bragg Grating. Thus, each pulse is reflected into two pulses that are separated by a short time interval. These parameters determine the location and wavelength shift of each individual Fiber Bragg Grating,
- optical frequency domain reflector (OFDR): multiple Fiber Bragg Gratings with almost identical central wavelength in a single core fiber. The sensor signal is generated by an adjustable laser source that sweeps a predetermined range of optical frequencies and is triggered by a Transistor-Transistor Logic (TTL) source. Two interferometers are used to obtain the sensor signal, one with the actual fiber in it and a second auxiliary to compensate for nonlinear frequency sweep in order to get a spatial resolution in the millimeter range. Both interferometers have to be measured simultaneously. In addition, the non-auxiliary interferometer also includes a reference arm with a single reflector. The acquired signal is then transferred into frequency domain and analyzed in order to obtain amplitude and phase information of each Fiber Bragg Grating.

The best suited multiplexing method depends on the task at hand. Nevertheless, for several reasons WDM is used if possible. While OTDR, OFDR, FSI, and TDM need sophisticated and partly highly expensive setups, WDM can be utilized with a single broadband source and an OSA. Additionally, spatial distance between individual sensing fields can be very low up to millimeters in contrast to other multiplexing approaches. Because of the cost-effectiveness of the interrogation setup as well as high spatial resolution, WDM is used in this work and covered with regard of performance and limitations in the parts below. Two main limitations of WDM are wavelength cross-talk, which limits the maximum number of Fiber Bragg Gratings per fiber, and spatial cross-talk, which limits the maximum spatial resolution of a sensor array with multiple Fiber Bragg Gratings. Wavelength cross-talk occurs, whenever the central wavelength separation between two Fiber Bragg Gratings is not sufficiently high. The signal of both Fiber Bragg Gratings overlaps in certain regions of the reflected spectrum which may lead to challenges in signal processing. This limits the maximum number of Fiber Bragg Gratings with respect to the spectral resolution of the OSA that in a typical setup is at optical c-band, as well as required signal separation. For instance, if the central wavelength of individual Fiber Bragg Grating is separated by $\pm 5$ nm across the optical c-band, only 6 Fiber Bragg Gratings fit into a single fiber. Separation must typically be a few nm when merely central wavelengths are of interest and only small central wavelength-shifts are expected. If, however, other signal parameters (e.g. energy, FWHM (full width half maximum), SLSR and so on) need to be retrieved and/or higher central wavelength-shifts are expected, the separation between central wavelengths must be substantially higher. On the other hand, spatial cross-talk can occur between Fiber Bragg Gratings with equal or different central wavelength. In theory, when multiple weak Fiber Bragg Gratings are put in an array, a multiple reflection paths arise. This is due to the weak nature of each individual Fiber Bragg Grating in the array, letting most of the signal energy passing through it. That in turn allows for reflection at a subsequent Fiber Bragg Grating. Dependent on the strength, the similarity and spatial distribution of Fiber Bragg Gratings multiple reflections may generate peaks with various central wavelengths that disturb the signal.

According to an embodiment of the invention, an optical measurement device for enabling simultaneous measurement of temperature, pressure and bending, is provided.

The optical measurement device comprises a (detachable) broadband light source Q, a fiber F, and a (detachable) optical spectrum analyzer OSA. That is only the fiber F - when used in medicine - may be needs to be sterilized. Also, having a detachable broadband light source Q and a detachable) optical spectrum analyzer OSA allows for using different fiber according to the present need.

Hence, if the costs for producing the fiber is low, it may even be a single use item.

In the fiber F at least two Fiber Bragg Gratings as a Fabry Pero-Interferometer are arranged allowing for reflection of light emitted by the light source Q into the fiber F, back through the fiber F towards the optical spectrum analyzer OSA.

Although not shown an optical isolator may be arranged such that light being emitted by the source is only directed towards the fiber F while light reflected in the fiber F is only directed towards the optical spectrum analyzer OSA.

Also, it may be envisaged to only provide short pulses of light into the fiber F whereas the OSA is only operating in times where only reflected light from the fiber F is to be expected depending on the length of the fiber F.

A reflection is depending of the temperature, pressure and bending applied to the fiber F in the region of the Fiber Bragg Grating Fabry Pero-Interferometer, whereby by use of the at least two Fiber Bragg Gratings as a Fabry-Perot Interferometer additional correlation features are produced allowing for separation of temperature, pressure and bending.

Unlike conventional approaches, the introduction of two Fiber Bragg Gratings as a Fabry-Perot Interferometer at first sight adds complexity as there is an interdependence of temperature, pressure and bending leading to intercoupling of these effects. However, unlike previous approaches to simplify the solution adding complexity allows for decomposing the effects as by the FBGFPI further information is created, particularly by choosing the reflectivity of the two Fiber Bragg Gratings as $R1 = R2*(1-R1)$ or at least closed to this relation, as explained previously.

Hence, by adding complexity decomposition is enabled.

To facilitate decomposition of temperature, pressure and bending effects and allow for determination at a same time, procedures may be used detailed later on.

According to an embodiment of the invention the fiber F is a single-mode fiber, in particular a single-mode fiber based on silicon dioxide.

According to yet another embodiment of the invention the fiber F is surrounded by a polymer, in particular polyimide or acrylate.

Polyimide and acrylate are both very popular coating polymers. Polyimide is preferred in high temperature environments due to its higher temperature resistance of up to 300 °C, compared to 100 °C for acrylate.

The selection of a coating may be based on other considerations as well. For example, a polyacrylate coating tends to be less stiff in comparison to a polyimide based coating.

Polymers, in particular polyimide as well as acrylates offer biocompatibility while they could also be sterilized in conventional processes.

According to an embodiment of the invention the fiber F is surrounded by a biocompatible material. This allows for example use in medicine.

Biocompatible polymers include polystyrene (PS), polypropylene (PP), polyvinyl chloride (PVC), polyethylene (PE), polyurethane (PU), polycarbonate (PC), polyethylene terephthalate (PET), polyetheretherketone (PEEK), polyacrylate, polyimide.

As stated before, dimensioning may be subject to certain constraints imposed by the usage.

In embodiments of the invention the fiber F comprises a core having a diameter of about 9.8 micrometer or less, in particular 9 micrometer or less, in particular 6.4 micrometer or less.

For example, a SM1250BI(9.8 / 125)P fiber having a core of 9.8 $\mu$m, a cladding of 125 $\mu$m and a coating of 155 $\mu$m made of polyimide may be used.

Another example is a SM1250SC(10/125)P fiber having a core of 9 $\mu$m, a cladding of 125 $\mu$m and a coating of 155 $\mu$m made of Polyimide may be used.

The gratings center may be located at different positions. In experiments, the center position of the gratings was located between 80 and 205 mm from the tip having a grating length of 3.9 mm.

A still further example is a SM1500(6.4/80)HT having a core of 6.4 $\mu$m, a cladding of 80 $\mu$m and a coating of 170 $\mu$m made of Acrylate may be used.

The gratings center may be located at different positions. In experiments, the center position of the gratings was located between 80 and 205 mm from the tip having a grating length of 3.0 mm.

According to an aspect of the invention the distance of two consecutive Fiber Bragg Gratings is approximately 3 mm.

Furthermore, in another aspect of the invention a distance between a first Fiber Bragg Grating element and a second Fiber Bragg Grating element may be chosen to provide differential data, such as the first Fiber Bragg Grating element being located close to the tip while the second Fiber Bragg Grating element is located such that when in use the second Fiber Bragg Grating element is at a location distal thereto, e.g. where it is to be expected that the fiber F experiences less or no bending when in use, in other words at least at the beginning of the elastically deformed region. This allows for identifying energy stored in the fiber F which might impact a patient.

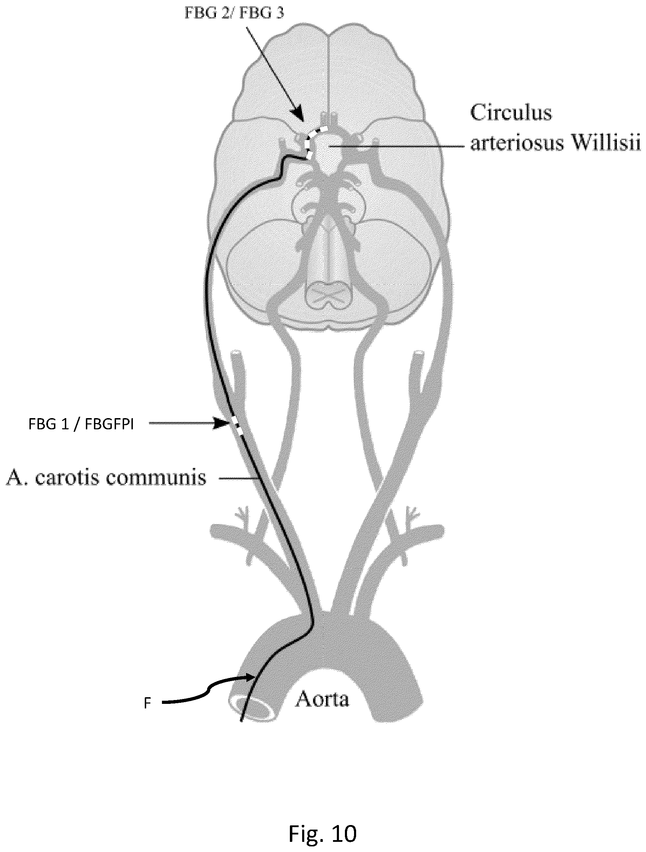

In Fig. 10 a fiber F is shown in relation to several locations of Fiber Bragg Gratings FBG1, FBG2, FBG3. Also shown are typical locations when used in a Circulus arteriosus Willisii, which is a typical site where aneurysm occur and may need treatment. The sensing elements allow detecting preloading along its longitudinal axis which may be required for the application as pusher wire. The FBGFPI is located in a (most) proximal location as shown in Figure 10. The two Fiber Bragg Gratings FBG2, FBG3 are located in a (most) distal location, separated e.g. by 1.5 mm.

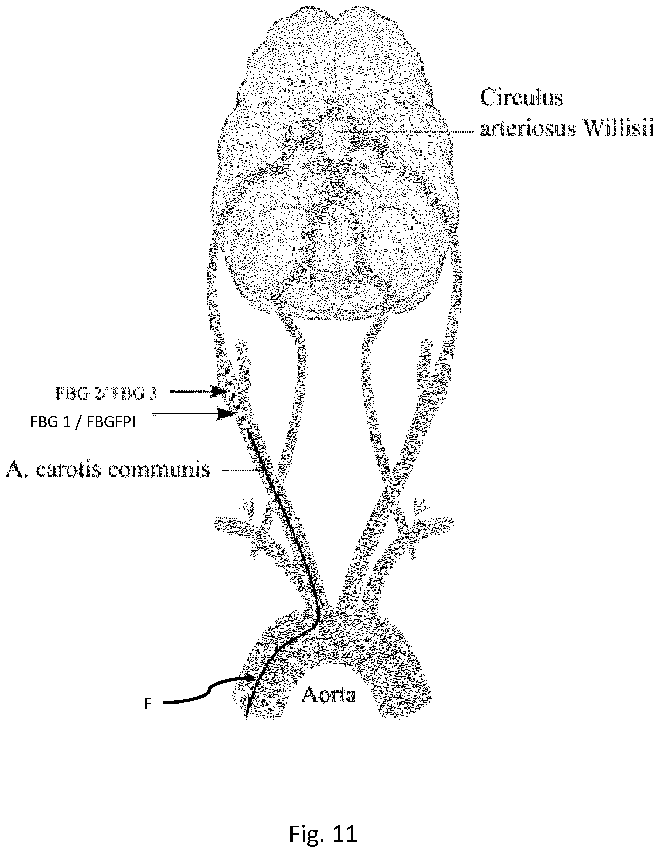

In Fig. 11, a like arrangement is shown for an intended uses as a guidewire. In this case it is not necessary to detect preload. Therefore, the sensing elements may be placed in close vicinity. To achieve the highest spatial sensing element density the sensing elements are separated by a small amount, e.g. 1.5 mm. Furthermore, with this configuration it is very likely, that all sensing elements are loaded with the same axial pressure during navigation, surrounded by the same temperature and are equally bent. Hence, there is some sort of redundancy which may be used as a verification.

In the shown arrangement the first sensing element is intended to be used as reference pressure sensor for the preloading detection. For this purpose, the difference between the load in a proximal portion of the instrument is detected and compared to the load of a distal portion of the instrument. If the proximal sensing element detects high pressure and the distal low pressure, this indicates a pre-loading and elastic deformation energy is stored in the instrument. To realize this feature the distance between the first sensing element FBG1 and the second sensing element FBG2 is determined so that during coiling the first sensing element is located in a straight portion of the common carotid artery. At the same time the second sensing element FBG2 is located in the circulus arteriosus cerebri CAC. This way, the elastic deformation energy stored in the winded and bend portions in between the first and second sensing element can be detected. The distance between FBG2 and the third sensing element FBG3 may be set to a low value, e.g. 3mm, in order to have two sensing elements that are in the same pressure, temperature and bending environment.

According to embodiments of the invention the central wavelengths of the gratings is within the optical c-band. If the center wavelengths are selected appropriately, one may cover the (most of the) optical c-band. This allows to benefit from dispersion effects in the respective sensor signal.

E.g. in embodiments a first Fiber Bragg Grating may have a central wavelength 1550 nm and the central wavelengths of a second Fiber Bragg Grating is 1560 nm. To provide an even better overview a third Fiber Bragg Grating may have a central wavelength 1535 nm.

In embodiments of the invention the distance between two Fiber Bragg Gratings having the same central wavelength is approximately in between 5.99 Millimeter and 7.99 Millimeter.

For example, a superstructure may be inscribed into one of the sensing elements. The goal is to add more information to the spectral response by generating transmission bands within the reflection band of the first sensing element.

The fundamental design is based on a Fiber Bragg Grating Fabry-Perot Interferometer (FBGFPI). The number of transmission bands, that occur due to the FPI portion of an FBGFPI, depend on the design of the Fiber Bragg Grating arrangement.

In order to analyze both variants, two sensors were exemplarily developed, whereby the number of transmission bands is chosen to be even in the first variant and odd for the second. By this measure, the central wavelength of the Fiber Bragg Grating remains reflective for the first sensor and become a transmission band for the second, generating two equally strong reflection signals at both sides.

The SFBG is generated by two Fiber Bragg Gratings that both have a central wavelength of 1535 nm. The first Fiber Bragg Grating - seen from the perspective of an incoming light beam - may offer a reflectivity of R = 45 %, in contrast to regular FBGFPI arrangements that utilize two highly reflective Fiber Bragg Gratings. This is done to allow for more light to propagate to further Fiber Bragg Gratings, e.g. a second Fiber Bragg Grating, which may offer a reflectivity of R = 75 % allowing for generating less aggressive transmission bands, in contrast to Gires-Tournois filters that use a combination of a low and very high mirrors or Fiber-Bragg-Grating. The SFBG that was generated offers transmission bands that only diminish the reflectivity at certain points. Although this property is substantially different from what is known from literature, in principle the structure is the same as FBGFPI. Therefore, the SFBG of this work is denoted as FBGFPI.

Exemplary sensors are designed to have three and six subpeaks. The grating parameters are calculated according to

$$\mathbb{T} = \frac{I_{out}}{I_{in}} = \left|\frac{E_{out}}{E_{in}}\right|^2 = \frac{1}{1 + \frac{4R}{(1-R)^2}\left(\sin\left(\frac{2\pi n}{\lambda}L_{FP}\right)\right)^2}$$

The center to center distance between the two Fiber Bragg Gratings that generate the FBGFPI was exemplarily determined to be $L_{FP}$ = 5.99 mm, respectively $L_{FP}$ = 7.99 mm. These positions of the gratings are determined with respect to the two application types pusher and guidewire.

The FBGFPI is located in the most proximal location of this arrangement. The two Fiber Bragg Gratings are located in the most distal location, separated by 1.5 mm.

For example, a SM1500BI(6.4/80)HT fiber having a core of 6.4 $\mu$m, a cladding of 80 $\mu$m and a coating of 170 $\mu$m made of polyimide may be used.

The gratings center may be located at different positions. In experiments, the center position of the gratings was located between 80 and 210 mm from the tip having a grating length of 3.0 mm (4 gratings located at 210, 205, 87 and 80 mm). In another experiment, the center position of the gratings was located between 483.5 and 500 mm from the tip having a grating length of 4.0 mm (4 gratings located at 500, 494.5 489 and 483.5 mm).

According to yet another aspect of the invention the light source is a superluminescent diode (SLD). These light sources offer the ability to produce light which covers a respective band, e.g. the optical c-band. For example, one may use a S5FC1005S- SM Benchtop SLD Source, available by Thorlabs GmbH Germany, offering a 1550 nm central wavelength, 22 mW, 50 nm bandwidth. Other light sources having higher power may be used as well.

In yet another aspect of the invention the optical measurement device further comprises a data processing entity. A data processing entity may be embodied in any suitable microcontroller, microprocessor, ASIC, FPGA, or the like.

According to an aspect of the invention the data processing entity is based on machine learning and/or deep learning.

I.e. by use of machine learning and/or deep learning evaluation may be simplified, allowing for quick and reliable results.

In embodiments of the invention the data processing entity is based on machine learning selected from the group comprising decision trees, support vector machines, Gaussian Process Regressions (GPRs), ensemble of bagged trees (EDTs), and artificial neural networks (ANNs).

Alternatively, in embodiments of the invention the data processing entity is based on deep learning selected from the group comprising multi-layer perceptrons (MLPs), gated recurrent units (GRUs), and long short-term memories (LSTMs).

The design approach that was used in this invention can be considered as top-down development. Hence, the system is developed based on requirements and constrains. As a consequence, the resulting design is highly adapted to the use case.

In the case of the TacFiber, the main goal is to give surgeons more insight about the force, that is applied by the tip of the instrument during the coiling procedure. This way Intraoperative Rupture are supposed to be prevented. Because of the thin and fragile nature of cranial vasculature and other constrains during the coiling procedure there are strict requirements with regard to dimensions and mechanic properties of TacFiber. In addition, there are performance requirements with respect to avoidance of intraoperative rupture both as pusher and guidewire. Finally, to be usable in endovascular coiling of intracranial aneurysms the system shall fulfill procedure related requirements.

As introduced above internal carotid arterys are predominantly prevalent in the circulus arteriosus cerebri. The thinnest artery that is part of the circulus arteriosus cerebri is the anterior communicating arteries with a diameter of only 1 mm.

However, the diameter of the largest micro catheters used in that region does not exceed 2.96 French. The most curved artery in the circulus arteriosus cerebri is the internal carotid artery with a bending radius of about 5 mm.

Therefore, the device according to the invention is designed to offer a diameter smaller 2 French and be bendable below a 5 mm radius. Also, both instruments shall be at least 2 m long to be able to reach cranial vessels from the regio inguinalis.

The primary function of the guidewire is to navigate the catheter and micro-catheter through the path from the femoral artery to the intracranial aneurysm. Complications arising from guidewires are rare but possible, such as vessel perforation due to excessive force when the guidewire is stuck in a bifurcation or bend. Gaining insights about the current tip bending and forces acting on the guidewire tip may help prevent these infrequent complications. Consequently, a force and bending sensor should be situated at the instrument's tip. Considering the vessel anatomy leading to common intracranial aneurysm locations, bending within a range of 5 - 15, mm must be accounted for. To enable longitudinal axis rotation for navigation purposes, the instrument must possess high torsional rigidity.

Intraoperative ruptures (IORs) occur due to several reasons. TacFiber aims to prevent force-related intraoperative rupture s by detecting inappropriately high propagation forces and stored elastic deformation energy. Therefore, a minimal setup requires at least two pressure sensing units within the device. One unit shall be near the tip to detect forces at that location. To identify energy stored in the instrument, a second force sensing unit should be placed at the beginning of the elastically deformed region, with the force difference indicating stored energy. To calculate the energy stored in the entire instrument, the second force sensor should be located at the instrument's beginning, where the force is introduced. However, in practice, the energy stored in the last segment of the instrument is of interest. The tortuous portions of the circulus arteriosus cerebri and the bending in the carotid canal are key contributors to this effect, as the path from the femoral artery to this region is relatively straight. Consequently, the second force sensor shall be placed proximal to the carotid canal. The internal carotid artery is suitable for this purpose, being the first straight part of the vasculature proximal to the carotid canal. Since the pusher is used to insert the coil through a previously positioned catheter, high torsion rigidity is unnecessary for navigation purposes. However, during coil administration, surgeons may wish to turn the pusher to influence the winding of the unfolding coils within the intracranial aneurysm. Thus, torsion rigidity should be in the medium to high range.

The force measuring range and resolution shall be chosen according to the forces acting on vessels during surgery. Unfortunately, no literature values exist for these forces in endovascular intracranial coiling procedures. Thus, a reverse engineering approach utilizing the physical properties of commonly used instruments is employed. Accordingly, the maximum exerted force is typically less than 53.1 mN for a commonly used guidewire as well as 45.1 mN, and 26.5, mN for commonly used coils. Consequently, the force sensor shall possess a resolution substantially smaller than 26.5 mN. This force value should be converted into a corresponding pressure, contingent upon the cross-sectional area of the TacFiber.

Besides, both guidewire and pusher are exposed to systolic and diastolic blood pressure, which periodically alters hydrostatic pressure and affects force measurements. In the internal carotid artery, the blood pressure varies between 77 mmHg (diastolic normotension) and 190 mmHg (systolic hypertension), equivalent to 10.26 kPa and 25.33 kPa, respectively. These values decrease further to 7.19 kPa and 19.33 kPa for the posterior parietal branch of the middle cerebral artery. Hence, taking into account both normotensive and hypertensive individuals, this pressure range is considerably lower than the tip pressure, but within a comparable order of magnitude, and thus cannot be completely disregarded. To measure the effect of blood pressure on measurements, the resolution shall be below the lowest difference between systolic and diastolic pressure, which corresponds to 31 mmHg or 4.13 kPa for the posterior parietal branch of the middle cerebral artery for a normotensive patient. However, since TacFiber primarily aims to detect excessively high tip forces that may cause intraoperative rupture, achieving a resolution within the realm of the lowest blood pressure differences is not critical.

Additionally, assuming a coiling procedure lasts more than 60 minutes with no intraprocedural heating, body temperature may drop up to 1.6 °C. To detect intraprocedural hypothermia and provide surgeons with further patient insights, the instrument shall also measure temperature with an accuracy and resolution of 0.1 °C or lower within a range of 36.4 - 37.3 °C. This allows for the detection of even slight core temperature changes.

Minimally invasive endovascular coiling of intracranial aneurysms is performed with support of digital subtraction angiography which is a form of computed tomography angiography. Consequently, the system shall be suited for CT environments, thus it must be insensitive to X-Rays. In the same way, MRI suitability and insensitivity to strong magnetic fields is demanded, in prospect of future developments in X-Ray free treatment procedures of intracranial aneurysms. The materials shall be suited for medical applications inside the human body and specifically intracranial application. Further, it is preferred that the system is mobile and can be operated outside of lab conditions. Also, the part of the system that is operated inside the human body and the part that is handled by the surgeon shall be easily sterilizable.

With the above detailed embodiments, it is possible to measure temperature, pressure and bending by a single device in one measurement.

In order to measure one needs to send at least a pulse of light by the source Q into the fiber F. Light being transmitted through the fiber F may then be reflected -at least in portion- by a respective Fiber Bragg Grating. I.e. as the reflection of a Fiber Bragg Grating will not be perfect, light will be transmitted via a respective Fiber Bragg Grating and interact in the same manner with a following Fiber Bragg Grating.

The sum of reflected light being subject to interaction with the Fiber Bragg Gratings inside the fiber F is then outputted again and fed towards an optical spectrum analyzer OSA. The optical spectrum analyzer OSA provides data pertaining to power at certain wavelengths.

In an embodiment the data provided by the optical spectrum analyzer OSA is evaluated by means of artificial intelligence.

A main idea behind ML is to generate an accurate prediction model that is able to learn from data. This is particularly useful, whenever the math behind a problem is too complex or (partially) unknown. Learning is achieved by a step during the model generation in which preliminary model outputs are compared with known true outputs. The prediction error is then computed as a function of the model parameters that is referred to as cost-function. In the subsequent steps, the cost function is then minimized to find the optimal model parameters to approximate the system behavior.

Since the term ML is used very loose nowadays, here a clear distinction is made and ML is solely used to refer to traditional ML. These include regression methods, support vector machines, ensemble methods and decision trees, and shallow neural networks that require manual feature extraction and selection. In contrast, the term DL, is used to refer to deep neural networks with more hidden layers.

According to the objective and scope of this invention prediction of continuous values is considered. Therefore, this section is focused on regression models that are trained by supervised learning, which means that the dataset is labeled.

To set up a proper ML model well-prepared data is paramount. An optimal dataset shall only include relevant linear independent signal features, whereas irrelevant features decrease the quality of the dataset. The process of finding and extracting features is known as feature extraction and requires sophistication and rigorous data analysis and domain knowledge.

A subset of regression models is listed below:

- Linear regression models (LR) and generalized linear regression models (GLMs)

- Support vector regression (SVRs)

- Ensemble models

- Decision trees (DT)

These models were selected to encompass all traditional ML methods in their most fundamental form.

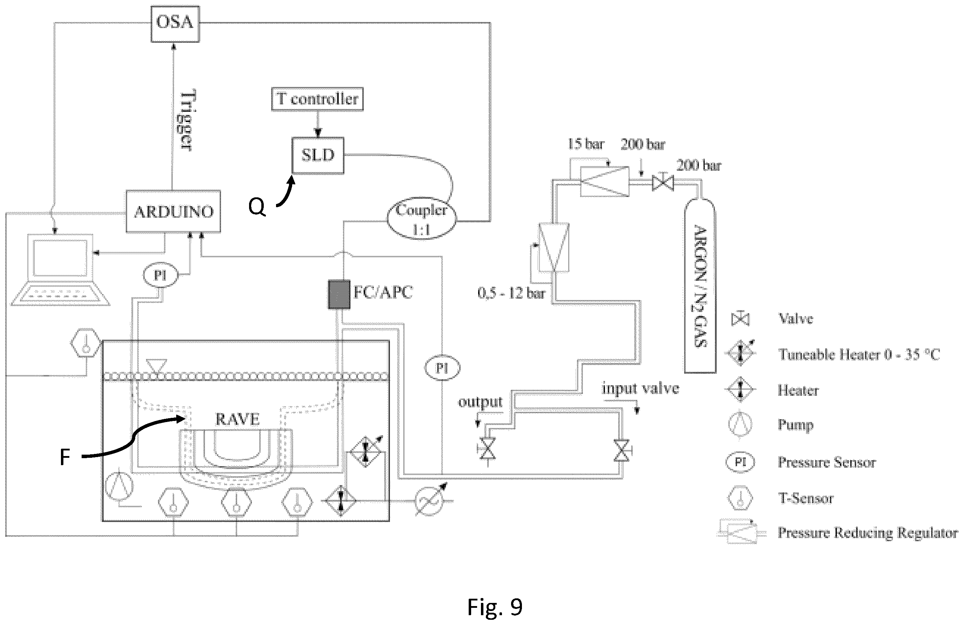

To help evaluate and / or train recognition by Machine Learning / Depp Learning an optical measurement device may be used in a defined setup as shown in Fig. 9. There an evaluation device shown as a laptop is receiving data measured by the OSA and data measured by other sensors allowing to precisely measure temperature, pressure with respect to a known bending. A known bending may be achieved by inserting a fiber F to be measured into a RAVE element as shown in Fig. 8 having predetermined radius. For evaluation the fiber F with its sensing portion may be subjected to different temperatures as well as different pressure. The bending may be changed by inserting the fiber F in another channel offered in the RAVE element. To allow for a quick data acquisition pressure and temperature may be steered by an appropriate steering device which could be provided by the evaluation device or as shown in Fig. 9 by a lightweight Microcontroller, such as an Ardurino.

In an aspect of the invention the evaluation is based on machine learning and/or deep learning.

In particular the data processing entity may be based on machine learning selected from the group comprising decision trees, support vector machines, Gaussian Process Regressions (GPRs), ensemble of bagged trees (EDTs), and artificial neural networks (ANNs).

Alternatively, the data processing entity may be based on deep learning selected from the group comprising multi-layer perceptrons (MLPs), gated recurrent units (GRUs), and long short-term memories (LSTMs).

It is noted that the invention and in particular the Fiber Bragg Grating Fabry Pero-Interferometer arrangement within a fiber F may be used in medicine.

**Claims**

1. An optical measurement device for enabling simultaneous measurement of temperature, pressure and bending, comprising a broadband light source (Q), a fiber (F), and an optical spectrum analyzer (OSA), whereby in the fiber (F) at least two Fiber Bragg Gratings as a Fabry-Perot Interferometer are arranged allowing for reflection of light emitted by the light source (Q) into the fiber (F), back through the fiber (F) towards the optical spectrum analyzer (OSA), whereby reflection is depending of the temperature, pressure and bending applied to the fiber (F) in the region of the Fiber Bragg Grating Fabry Perot Interferometer, whereby by use of the at least two Fiber Bragg Gratings additional correlation features are produced allowing for separation of temperature, pressure and bending.

2. The optical measurement device according to claim 1, whereby the fiber (F) is a single-mode fiber based on silicon dioxide.

3. The optical measurement device according to claim 1 or 2, whereby the fiber (F) is surrounded by a polymer, in particular polyimide or acrylate.

4. The optical measurement device according to any preceding claim, whereby the light source is a superluminescent diode.

5. The optical measurement device according to any preceding claim, further comprising a data processing entity.

6. The optical measurement device according to claim 5, whereby the data processing entity is based on machine learning and/or deep learning.

7. The optical measurement device according to claim 5, whereby the data processing entity is based on machine learning selected from the group comprising decision trees, support vector machines, Gaussian Process Regressions (GPRs), ensemble of bagged trees (EDTs), and artificial neural networks (ANNs).

8. The optical measurement device according to claim 5, whereby the data processing entity is based on deep learning selected from the group comprising multi-layer perceptrons (MLPs), gated recurrent units (GRUs), and long short-term memories (LSTMs).

9. The optical measuring device according to one of the preceding claims, whereby the reflectivity of a first Fiber Bragg Grating and a second Fiber Bragg Gratings is selected such that the power being reflected by the second Fiber Bragg Grating after having been transmitted via the first Fiber Bragg Grating is within the same scale as the reflected power from the first Fiber Bragg Grating.

10. Use of a Fiber Bragg Grating Fabry-Perot Interferometer according to any preceding claim in medicine.

11. Method for measuring temperature, pressure and bending by a Fiber Bragg Grating Fabry-Perot Interferometer according to any preceding claim 1 to 9.

12. The method according to claim 11, whereby the data provided by the optical spectrum analyzer (OSA) is evaluated by means of artificial intelligence.

13. The method according to claim 11 or 12, whereby the evaluation is based on machine learning and/or deep learning.

14. The method according to claim 13, whereby the data processing entity is based on machine learning selected from the group comprising decision trees, support vector machines, Gaussian Process Regressions (GPRs), ensemble of bagged trees (EDTs), and artificial neural networks (ANNs).

15. The method according to claim 13, whereby the data processing entity is based on deep learning selected from the group comprising multi-layer perceptrons (MLPs), gated recurrent units (GRUs), and long short-term memories (LSTMs).

16. Use of a method according to one of claims 11 to 15 in medicine.

Fig. 1a

Fig. 1b

Fig. 2

Transmitted

I

Source

$\lambda_{[nm]}$

$\lambda_B$

Fig. 3a

Reflected

I

Source

$\lambda$

$\lambda_B$

Fig. 3b

$\bar{\bar{n}}$

$n_{eff}$

$n$

$\Delta x_n$

$\Lambda_G$

Fig. 4

1) Uniform Positive-Only Index Change

2) Gaussian-Apodized Index Change

3) Raised-Cosine-Apodized Zero-dc Index Change

4) Discrete Phase Shift Index Change

(A) Grating types.

(B) Corresponding index modulations.

Fig. 5

A.I)

B.I)

C.I)

A.II)

B.II)

C.II)

Fig. 6

Fig. 7

RAVE

Fig. 8

Fig. 9

Fig. 10

Fig. 11

**EP 4 636 362 A1**

| Europäisches Patentamt<br>European Patent Office<br>Office européen des brevets | **EUROPEAN SEARCH REPORT** | **Application Number**<br>EP 25 17 0689 |
| --- | --- | --- |

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| X | Shojaei Khatouni Sohrab ET AL: "Discriminating Temperature, Strain, and Bending Effects in Fiber Bragg Grating Fabry- Perot Interferometer Sensors Using Traditional Machine Learning Techniques", TechRxiv, 19 April 2023 (2023-04-19), XP093291908, DOI: 10.36227/techrxiv.22644031.v1 Retrieved from the Internet: URL:https://www.techrxiv.org/doi/full/10.36227/techrxiv.22644031.v1 [retrieved on 2025-07-02] * the whole document * | 1-16 | INV.<br>G01D5/353 |
| A | PEVEC SIMON ET AL: "MultiParameter Fiber-Optic Sensor for Simultaneous Measurement of Thermal Conductivity, Pressure, Refractive Index, and Temperature", IEEE PHOTONICS JOURNAL, vol. 9, no. 1, 1 February 2017 (2017-02-01), pages 1-14, XP093292051, USA ISSN: 1943-0655, DOI: 10.1109/JPHOT.2017.2651978 * 1. Introduction 2. Sensor design and operation * * figures 1,4 * | 1-16 | |
| A | US 2018/364073 A1 (ALEMOHAMMAD HAMID [CA] ET AL) 20 December 2018 (2018-12-20) * paragraph [0016] - paragraph [0031] * * figures 1-2 * | 1-16 | TECHNICAL FIELDS SEARCHED (IPC)<br><br>G01D |

-/--

The present search report has been drawn up for all claims

| Place of search | Date of completion of the search | Examiner |
| --- | --- | --- |
| Munich | 2 September 2025 | Paraf, Edouard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

| | Europäisches Patentamt European Patent Office Office européen des brevets | **EUROPEAN SEARCH REPORT** | **Application Number** EP 25 17 0689 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ZHANG SHENGQI ET AL: "Simultaneous Measurement of Pressure, Temperature and Salinity Based on Tilted Fiber Bragg Grating and Fabry-Perot Interferometer for Marine Monitoring", 2022 ASIA COMMUNICATIONS AND PHOTONICS CONFERENCE (ACP), IEEE, 5 November 2022 (2022-11-05), pages 160-165, XP034327383, DOI: 10.1109/ACP55869.2022.10088696 [retrieved on 2023-04-10] * III. Experimental result and discussion IV. Conclusion * ----- | 1-16 | |
| | | | **TECHNICAL FIELDS SEARCHED (IPC)** |

The present search report has been drawn up for all claims

| Place of search | Date of completion of the search | Examiner |
|---|---|---|
| Munich | 2 September 2025 | Paraf, Edouard |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

**ANNEX TO THE EUROPEAN SEARCH REPORT
ON EUROPEAN PATENT APPLICATION NO.**                    EP 25 17 0689

This annex lists the patent family members relating to the patent documents cited in the above-mentioned European search report.
The members are as contained in the European Patent Office EDP file on
The European Patent Office is in no way liable for these particulars which are merely given for the purpose of information.

02-09-2025

| Patent document cited in search report | Publication date | Patent family member(s) | Publication date |
|---|---|---|---|
| US 2018364073 A1 | 20-12-2018 | CA 3066288 A1 | 20-12-2018 |
| | | CA 3208719 A1 | 20-12-2018 |
| | | US 2018364073 A1 | 20-12-2018 |
| | | US 2021116266 A1 | 22-04-2021 |
| | | WO 2018227281 A1 | 20-12-2018 |

EPO FORM P0459

For more details about this annex : see Official Journal of the European Patent Office, No. 12/82